# EUROPEAN PATENT APPLICATION

(11) **EP 4 686 445 A1**
(43) Date of publication of application: **04.02.2026**
(21) Application number: 25182782.0
(22) Date of filing: 13.06.2025
(51) Int. Cl.: A61B 3/00, A61B 3/14

(54) **OPHTHALMIC APPARATUS, METHOD FOR GENERATING HIGH DYNAMIC-RANGE IMAGE FOR OPHTHALMIC APPARATUS, AND PROGRAM FOR GENERATING HIGH DYNAMIC-RANGE IMAGE FOR OPHTHALMIC APPARATUS**

(30) Priority: 29.07.2024 JP 2024122599
(71) Applicant: Takagi Seiko Co., Ltd., Nakano-shi, Nagano 383-8585 (JP)
(72) Inventor: NAGAI, Kazuki, Nakano-shi, 383-8585 (JP)
(74) Representative: Mewburn Ellis LLP

(57) **Abstract**

An imaging means (10) for periodically taking test subject eye images for each different exposure condition, a storage means (20) for storing the eye images with capturing time information as imaging data, and the selected eye image from the imaging data as base image, and an arithmetic unit (50) for storing a copy of the base image in the storage means (20) as an output image, performing HDR processing on the output image to generate an HDR image, and determining identicalness of the eye image to the base image are provided, wherein the arithmetic unit (50) performs updating the output image by superimposing the eye image on the output image to store the output image in the storage means (20) on the eye images in the same exposure condition to generate the HDR image when the eye image and the base image are determined to be the same chronologically.

## Description

### Technical Field

The present invention relates to an ophthalmic apparatus, a method for generating a high dynamic-range image for an ophthalmic apparatus, and a program for generating a high dynamic-range image for an ophthalmic apparatus, and relates in particular to an ophthalmic apparatus capable of efficiently generating a high dynamic-range image from a photographic image, a method for generating the high dynamic-range image for the ophthalmic apparatus, and a program for generating the high dynamic-range image for the ophthalmic apparatus.

### Background Art

A technique for generating a high dynamic-range image is used in a variety of fields as processing for approximating an image taken by a camera or the like to an appearance viewed by a human. As such method for generating a high dynamic-range image, there is known such a technique as disclosed in PTL 1 (JP-A-2024-14009). In PTL 1, there is disclosed a technique for making an image to be used in an ophthalmic apparatus into a high dynamic-range image.

### Summary of Invention

### Technical Problem

The ophthalmic apparatus disclosed in PTL 1 is for generating the high dynamic-range image by combining images taken by an imaging device with each other, wherein it is assumed that the images taken by the imaging device are totally the same as each other except imaging conditions such as time of exposure. However, during imaging in the ophthalmic apparatus, an image at the moment of a blink may be included in some cases. It has been made known that there is a problem that when the high dynamic-range image is generated using such images different from each other, there occurs a trouble that a disturbance occurs in the image thus generated.

### Solution to Problem

Therefore, one of the objects of the present invention is to provide an ophthalmic apparatus capable of generating a high dynamic-range image free of disturbance even when a test subject makes a blink during imaging, a method for generating the high dynamic-range image for the ophthalmic apparatus, and a program for generating the high dynamic-range image for the ophthalmic apparatus.

As a result of an earnest study for solving the problems described above by the present inventors, the following configurations have been conceived. That is, the present invention is an ophthalmic apparatus including an imaging means configured to take test subject eye images at constant time intervals for each of exposure conditions different from each other, a storage means configured to store, as imaging data, the test subject eye image together with capturing time information, and store, as a base image, the test subject eye image selected by similarly to a base image selecting reference image or a gauger out of the test subject eye images in the imaging data, an output image initialization means configured to store a copy of the base image in the storage means as an output image, a high dynamic-range image generation means configured to perform high dynamic-range achieving processing on the output image to generate a high dynamic-range image, and an image identicalness determination means configured to determine whether or not the test subject eye image in the imaging data is same as the base image, wherein the high dynamic-range image generation means executes processing of updating the output image by superimposing the test subject eye image on the output image and storing the output image in the storage means on the test subject eye image in same exposure condition to generate the high dynamic-range image only when the image identicalness determination means compares similarity between the test subject eye image and the base image in a chronological order of the capturing time information, and determines that the test subject eye image and the base image are same as each other.

Thus, generation of a high dynamic-range image free of disturbance is made possible even when a test subject makes a blink during imaging.

Further, the high dynamic-range image generation means preferably executes processing of continuously using the output image when a difference between the capturing time information when the image identicalness determination means first determines that the test subject eye image is different from the base image and the capturing time information when the image identicalness determination means determines that the test subject eye image is restored to the base image is within a first threshold value set in advance, and preferably executes processing of making the imaging means take the test subject eye image once again, processing of storing, as the imaging data, the test subject eye image taken once again together with the capturing time information in the storage means, processing of storing, as the base image, the test subject eye image selected from the test subject eye images in the imaging data in the storage means, and processing of making the output image initialization means store a copy of the base image in the storage means as the output image when the difference between the capturing time information when the image identicalness determination means first determines that the test subject eye image is different from the base image and the capturing time information when the image identicalness determination means determines that the test subject eye image is restored to the base image exceeds the first threshold value set in advance.

Thus, when an image change due to a blink which is a physiological phenomenon occurs, since the images before and after the image change are substantially the same as each other in most cases, it is possible to use the image data before the image change without discarding the image data.

Further, it is preferable for the image identicalness determination means to execute processing of dividing each of the base image, the test subject eye image, and the output image into a plurality of divided areas same in shape and arrangement, processing of updating the output image by superimposing the divided areas in the test subject eye image on the divided areas in the output image when a difference in identicalness determining numerical value between the divided areas is no greater than a second threshold value set in advance in each of the divided areas of the test subject eye image and the base image at positions same as each other, and processing of keeping the output image when the difference in identicalness determining numerical value between the divided areas exceeds the second threshold value in each of the divided areas of the test subject eye image and the base image at positions same as each other.

Thus, even when it is determined that the image change has occurred as a whole of the image, the data corresponding to a portion in which the image change has not substantively occurred can be used.

Further, it is preferable that based on either one of at least one of values (an R value, a G value, and a B value) out of RGB values in each of pixels of the test subject eye image and the base image, or a conversion value obtained by converting the RGB values with a conversion formula set in advance, the identicalness determining numerical value is an average value of the values or the conversion values of the pixels in each of the divided areas.

Thus, it is possible to shorten the data processing time for determining the identicalness of the image.

Further, it is preferable to further include an image display control means configured to make the image display unit display at least one of the base image and the output image, and it is preferable for the image display control means to make the image display unit display the output image every time the output image is updated.

Thus, it is possible to confirm the image in the middle of the generation of the high dynamic-range image.

Further, there is also a method for generating a high dynamic-range image for an ophthalmic apparatus used for the ophthalmic apparatus equipped with an imaging means configured to take an eye image of a test subject, a storage means, and an arithmetic unit, the method including a step of making, by the arithmetic unit, the imaging means take test subject eye images at constant time intervals for each of exposure conditions different from each other, a step of making, by the arithmetic unit, the storage means store, as imaging data, the test subject eye image together with capturing time information, a step of making, by the arithmetic unit, the storage means store, as a base image, the test subject eye image which is selected based on similarity to a base image selecting reference image, or selected by a gauger, out of the test subject eye images in the imaging data, a step of making, by the arithmetic unit, the storage means store a copy of the base image as an output image, a step of performing, by the arithmetic unit, high dynamic-range achieving processing on the output image to generate a high dynamic-range image, and a step of determining, by the arithmetic unit, whether or not the test subject eye image in the imaging data is same as the base image, wherein the step of generating the high dynamic-range image includes a step of making the arithmetic unit compare similarity between the test subject eye image and the base image in a chronological order of the capturing time information, and then making the arithmetic unit update the output image by superimposing the test subject eye image on the output image and store the output image in the storage means only when it is determined that the test subject eye image and the base image are same as each other, and a step of comparing, by the arithmetic unit, the similarity between the test subject eye image and the base image in a chronological order of the capturing time information, and then making the arithmetic unit keep the output image when it is determined that the test subject eye image is different from the base image.

Thus, generation of a high dynamic-range image free of disturbance is made possible even when a test subject makes a blink during imaging.

Further, it is preferable to include a step of continuously using the output image when a difference between the capturing time information when the arithmetic unit first determines that the test subject eye image is different from the base image and the capturing time information when the arithmetic unit determines that the test subject eye image is restored to the base image is within a first threshold value set in advance, a step of making the imaging means take the test subject eye image once again when the difference between the capturing time information when the arithmetic unit first determines that the test subject eye image is different from the base image and the capturing time information when the arithmetic unit determines that the test subject eye image is restored to the base image exceeds the first threshold value set in advance, a step of making the storage means store, as imaging data, the test subject eye image taken once again together with the capturing time information, a step of storing, as the base image, the test subject eye image selected from the test subject eye images in the imaging data in the storage means, and a step of making the arithmetic unit store, as the output image, a copy of the base image in the storage means.

Thus, when an image change due to a blink which is a physiological phenomenon occurs, since the images before and after the image change are substantially the same as each other in most cases, it is possible to use the image data before the image change without discarding the image data.

Further, it is preferable to execute a step of dividing, by the arithmetic unit, each of the base image, the test subject eye image, and the output image into a plurality of divided areas same in shape and arrangement, a step of updating the output image by superimposing the divided areas in the test subject eye image on the divided areas in the output image when the arithmetic unit determines that a difference in identicalness determining numerical value between the divided areas is no greater than a second threshold value set in advance in each of the divided areas of the test subject eye image and the base image at positions same as each other, and a step of keeping the output image when the arithmetic unit determines that the difference in identicalness determining numerical value between the divided areas exceeds the second threshold value in each of the divided areas of the test subject eye image and the base image at positions same as each other.

Thus, even when it is determined that the image change has occurred as a whole of the image, the data corresponding to a portion in which the image change has not substantively occurred can be used.

Further, it is preferable that based on either one of at least one of values (an R value, a G value, and a B value) out of RGB values in each of pixels of the test subject eye image and the base image, or a conversion value obtained by converting the RGB values with a conversion formula set in advance, the arithmetic unit uses an average value of the values or the conversion values of the pixels in each of the divided areas as the identicalness determining numerical value.

Thus, it is possible to shorten the data processing time for determining the identicalness of the image.

Further, it is preferable for the ophthalmic apparatus to further include an image display unit configured to display at least one of the base image and the output image, and an image display control means configured to make the image display unit display at least one of the base image and the output image, and it is preferable to include a step of making, by the arithmetic unit, the image display unit display the output image every time the output image is updated.

Thus, it is possible to confirm the image in the middle of the generation of the high dynamic-range image.

Further, there is also a program for generating a high dynamic-range image for an ophthalmic apparatus used for the ophthalmic apparatus equipped with an imaging means configured to take an eye image of a test subject, a storage means, and an arithmetic unit, the program making the arithmetic unit execute processing of making the imaging means take test subject eye images at constant time intervals for each of exposure conditions different from each other, processing of making the storage means store, as imaging data, the test subject eye image together with capturing time information, processing of making the storage means store, as a base image, the test subject eye image which is selected based on similarity to a base image selecting reference image, or selected by a gauger, out of the test subject eye images in the imaging data, processing of storing a copy of the base image in the storage means as an output image, processing of generating a high dynamic-range image by performing high dynamic-range achieving processing on the output image, and processing of determining whether or not the test subject eye image in the imaging data is same as the base image, wherein the processing of generating the high dynamic-range image includes processing of comparing similarity between the test subject eye image and the base image in a chronological order of the capturing time information, and then updating the output image by superimposing the test subject eye image on the output image and storing the output image in the storage means when it is determined that the test subject eye image and the base image are same as each other, and processing of comparing the similarity between the test subject eye image and the base image in a chronological order of the capturing time information, and then keeping the output image when it is determined that the test subject eye image is different from the base image.

Thus, generation of a high dynamic-range image free of disturbance is made possible even when a test subject makes a blink during imaging.

Further, it is preferable to make the arithmetic unit execute processing of continuously using the output image when a difference between the capturing time information when it is first determined that the test subject eye image is different from the base image and the capturing time information when it is determined that the test subject eye image is restored to the base image is within a first threshold value set in advance, processing of making the imaging means take the test subject eye image once again when the difference between the capturing time information when it is first determined that the test subject eye image is different from the base image and the capturing time information when it is determined that the test subject eye image is restored to the base image exceeds the first threshold value set in advance, processing of making the storage means store, as the imaging data, the test subject eye image taken once again together with the capturing time information, processing of storing, as the base image, the test subject eye image selected from the test subject eye images in the imaging data in the storage means, and processing of storing a copy of the base image in the storage means as the output image.

Thus, when an image change due to a blink which is a physiological phenomenon occurs, since the images before and after the image change are substantially the same as each other in most cases, it is possible to use the image data before the image change without discarding the image data.

Further, it is preferable to make the arithmetic unit execute processing of dividing each of the base image, the test subject eye image, and the output image into a plurality of divided areas same in shape and arrangement, processing of updating the output image by superimposing the divided areas in the test subject eye image on the divided areas in the output image when it is determined that a difference in identicalness determining numerical value between the divided areas is no greater than a second threshold value set in advance in each of the divided areas of the test subject eye image and the base image at positions same as each other, and processing of keeping the output image when it is determined that the difference in identicalness determining numerical value between the divided areas exceeds the second threshold value in each of the divided areas of the test subject eye image and the base image at positions same as each other.

Thus, even when it is determined that the image change has occurred as a whole of the image, the data corresponding to a portion in which the image change has not substantively occurred can be used.

Further, it is preferable that based on either one of at least one of values (an R value, a G value, and a B value) out of RGB values in each of pixels of the test subject eye image and the base image, or a conversion value obtained by converting the RGB values with a conversion formula set in advance, the arithmetic unit is made to use an average value of the values or the conversion values of the pixels in each of the divided areas as the identicalness determining numerical value.

Thus, it is possible to shorten the data processing time for determining the identicalness of the image.

Further, it is preferable for the ophthalmic apparatus to further include an image display unit configured to display at least one of the base image and the output image, and an image display control means configured to make the image display unit display at least one of the base image and the output image, and it is preferable to make the arithmetic unit display the output image on the image display unit every time the output image is updated.

Thus, it is possible to confirm the image in the middle of the generation of the high dynamic-range image.

### Advantageous Effects of Invention

According to the configurations of the ophthalmic apparatus, the method for generating a high dynamic-range image for an ophthalmic apparatus, and the program for generating a high dynamic-range image for an ophthalmic apparatus in the present invention, it becomes possible to generate a high dynamic-range image free of disturbance even when a test subject makes a blink during imaging. Brief Description of Drawings
Fig. 1 is a schematic configuration diagram of an ophthalmic apparatus in the present embodiment.
Fig. 2 is a flowchart of overall processing of image data in the ophthalmic apparatus in the present embodiment.
Fig. 3 is a detailed flowchart of initialization processing in Fig. 2.
Fig. 4 is an explanatory diagram showing a specific example when dividing a present-image buffer N and a previous-frame-image buffer B by a change observation area AREA.
Fig. 5 is a detailed flowchart of imaging/digitalizing processing in Fig. 2.
Fig. 6 is a detailed flowchart of high dynamic-range image generation processing in Fig. 2.
Fig. 7 is an explanatory diagram showing a specific example of high dynamic-range image generation processing.
Fig. 8 is a detailed flowchart in a subroutine of blink determination processing in Fig. 6.
Fig. 9 is an explanatory diagram showing a specific example in the blink determination processing.
Fig. 10 is a detailed flowchart of output screen update/imaging condition setup processing in Fig. 2.
Fig. **11** is an explanatory diagram showing a specific example of an imaging condition (an exposure condition).

### Description of Embodiments

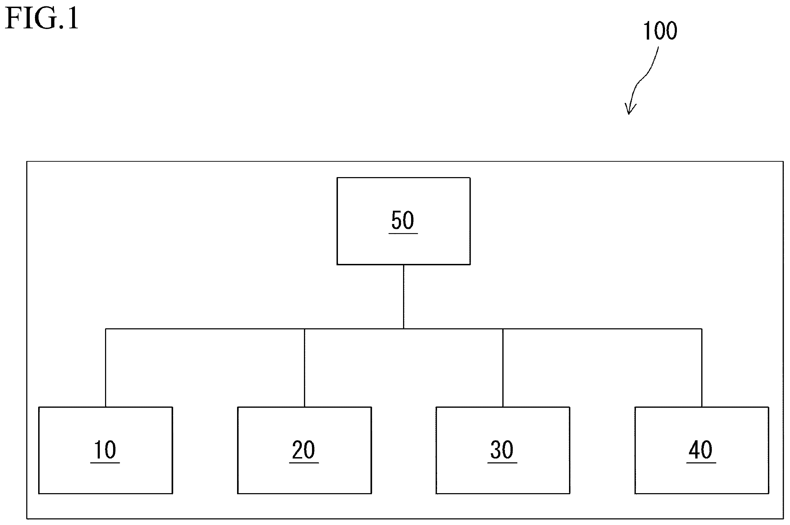

Hereinafter, an embodiment of an ophthalmic apparatus 100 according to the present invention will be described with reference to Fig. 1. The ophthalmic apparatus 100 in the present embodiment is provided with an imaging means 10, a storage means 20, an image display unit 30, an external input output unit 40, and an arithmetic unit 50. The arithmetic unit 50 controls each of operations of the imaging means 10, the storage means 20, the image display unit 30, and the external input output unit 40 based on an operation control program including a high dynamic-range image generation program stored in the storage means 20. Specifically, processing of making the imaging means 10 take test subject eye images at constant intervals for each of exposure conditions different from each other, processing of making the storage means 20 store the test subject eye image data thus taken, processing of making the arithmetic unit 50 generate a high dynamic-range image based on the test subject eye image data stored in the storage means 20, and processing of making the image display unit 30 output the high dynamic-range image thus generated are executed. It should be noted that it is preferable for the imaging means 10 to take the test subject eye photographic images at constant intervals of 30 to 60 frames per second for several seconds although depending on the imaging means 10 actually used.

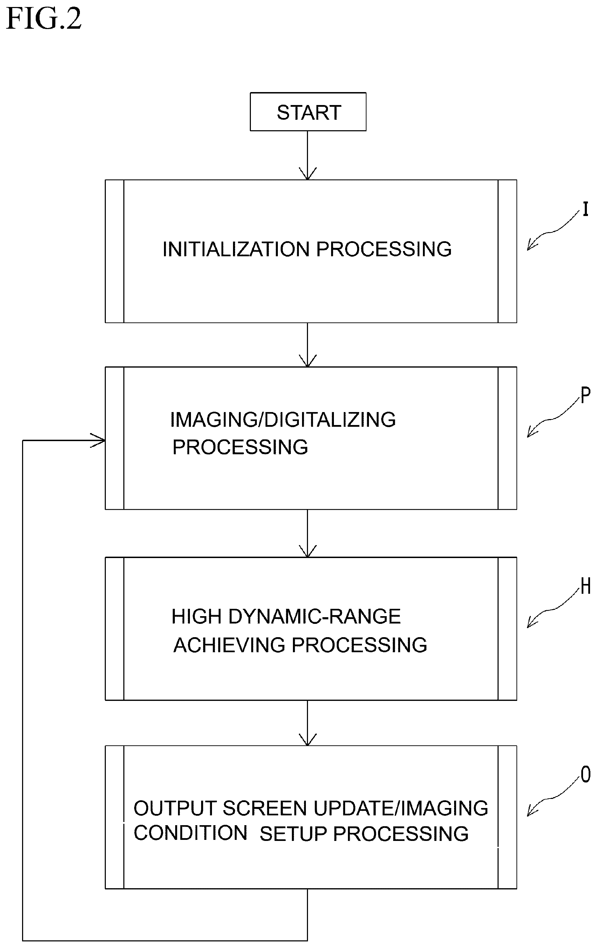

Then, processing content when the ophthalmic apparatus 100 in the present embodiment makes the test subject eye image into a high dynamic-range image will be described using Fig. 2 and so on. As shown in Fig. 2, in the ophthalmic apparatus 100 according to the present embodiment, there are performed four processing, that is, initialization processing I, imaging/digitalizing processing P, high dynamic-range achieving processing H, and output screen update/imaging condition setup processing O. Further, the imaging/digitalizing processing P, the high dynamic-range achieving processing H, and the output screen update/imaging condition setup processing O are repeatedly executed for each test subject or for each inspection item as a result.

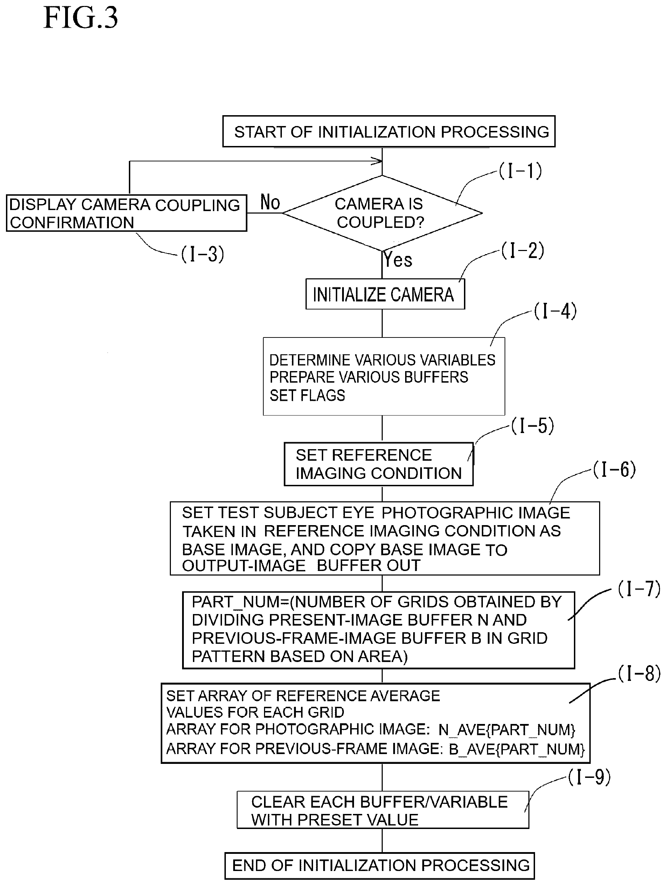

Then, specific processing content in the initialization processing I will be described using Fig. 3. When the ophthalmic apparatus 100 is started up by a gauger, the arithmetic unit 50 as an initialization means executes the initialization processing I. Specifically, the arithmetic unit 50 confirms (I-1) whether or not a camera as the imaging means 10 is coupled via the external input output unit 40 represented by a USB coupling terminal of the ophthalmic apparatus 100, and when the imaging means 10 is coupled, the arithmetic unit 50 executes processing (I-2) of initializing the imaging means 10 into an available state. On the other hand, when the imaging means 10 is not coupled when performing the processing (I-1), display processing (I-3) of confirming the coupling of the imaging means 10 is performed on the image display unit 30, and then the process returns to the processing (I-1).

Subsequently, the arithmetic unit 50 executes processing (I-4) of preparing a variety of types of data to be stored in the storage means 20. Specifically, processing of outputting a screen which prompts the gauger to input numerical values of a first change amount threshold value THR, a change observation area AREA, an area change threshold value AREA_THR, a blink determination reference time T, and a second change amount threshold value THR2 on the image display unit 30 is executed. The numerical values of the first change amount threshold value THR, the change observation area AREA, the area change threshold value AREA_THR, and the blink determination reference time T input from an input means which is not shown and is coupled via the external input output unit 40 by the gauger are each stored in the storage means 20. Further, the arithmetic unit 50 executes processing of preparing a present-image buffer N, a previous-frame-image buffer B, an output-image buffer OUT, a reference value buffer Y, a present image capturing time NT, a previous-frame image capturing time BT, a base-image buffer JB, base image time data JBT, a changed-image buffer JP, and a changed image capturing time JPT in the storage means 20. Further, the arithmetic unit 50 executes processing of setting an imaging restart flag to TRUE and storing the imaging restart flag in the storage means 20.

Subsequently, the gauger or the arithmetic unit 50 executes processing (I-5) of setting a reference imaging condition of the imaging means 10 for taking an eye photographic image of the test subject. Specifically, the exposure condition in which a so-called halation can narrowly be prevented from occurring in the test subject eye image is set as the reference imaging condition while displaying the test subject eye image, which is obtained by imaging the eye of the test subject with the imaging means 10, on the image display unit 30. In setting the reference imaging condition, it is possible to adopt a configuration in which the arithmetic unit 50 executes processing of setting appropriate reference imaging condition based on a reference imaging condition determining program stored in advance in the storage means 20 and an illuminance and a luminance of the test subject eye image taken by the imaging means 10. After the reference imaging condition is set, the arithmetic unit 50 executes processing (I-6) in which the imaging means 10 is made to take the test subject eye photographic image in the reference imaging condition, the test subject eye photographic image thus taken is set to the base image, and the arithmetic unit 50 as an output image initialization means stores the base image in the storage means 20 as an output image buffer OUT which is an output image.

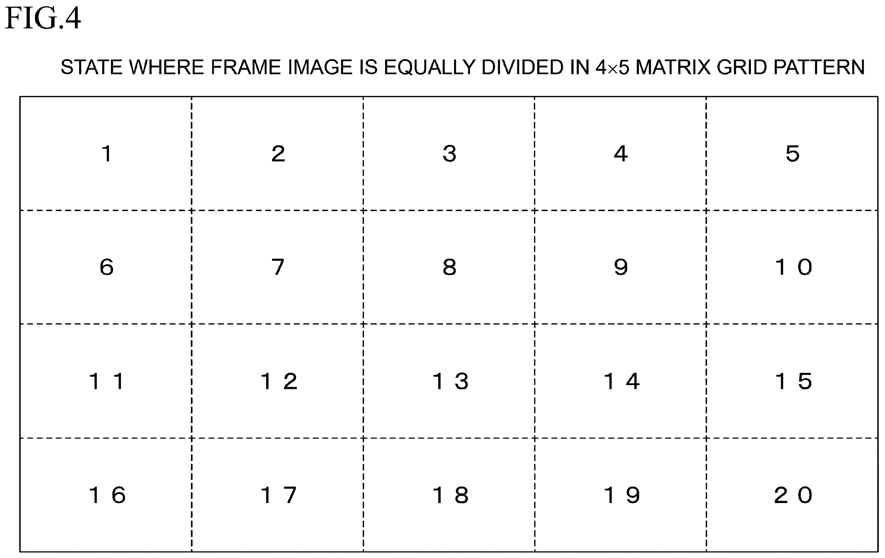

Subsequently, the arithmetic unit 50 as an image identicalness determination means executes processing (I-7) of dividing each of the present-image buffer N and the previous-frame-image buffer B in a grid pattern based on the change observation area AREA stored in the storage means 20. In the present-image buffer N, the test subject eye image (a frame image) taken by the imaging means 10 is stored, and in the previous-frame-image buffer B, the base image described later is stored first, and then, a previous-frame image in time series of the frame image of the test subject eye image stored in the present-image buffer N is stored sequentially. The arithmetic unit 50 divides each of the frame images stored in the present-image buffer N and the previous-frame-image buffer B into a plurality of grids for each of the change observation area AREA as shown in Fig. 4. Further, the arithmetic unit 50 performs processing of setting a number 1 to a grid at the left end position in the uppermost stage, then incrementing the number toward the grid at the right end position, and then providing serial numbers to all the grids in the subsequent stages in a similar rule. Here, in Fig. 4, a portion surrounded by solid lines is the frame image, and a portion surrounded by broken lines or broken lines and the solid line is the grid.

In the present embodiment, each of the present-image buffer N and the previous-frame-image buffer B is divided into 20 grids in a 4×5 matrix as shown in Fig. 4, but the division number of the grids is not particularly limited as long as the division number is a natural number. It should be noted that in the present embodiment, the arithmetic unit 50 also executes, in combination, processing of storing the number of grids in the storage means 20 as PART_NUM. It should be noted that the arithmetic unit 50 in the present embodiment divides each of the present-image buffer N and the previous-frame-image buffer B in the grid shape the same in shape and arrangement based on the change observation area AREA, but this configuration is not a limitation. It is possible to adopt a configuration in which the arithmetic unit 50 divides each of the present-image buffer N and the previous-frame-image buffer B in the grid shape based on image dividing data for dividing each of the present-image buffer N and the previous-frame-image buffer B in the grid shape of an m×n matrix which is stored in advance in the storage means 20, or a configuration in which the arithmetic unit 50 prompts the gauger to input image division numbers (m, n) via a data input means not shown, and the arithmetic unit 50 divides each of the present-image buffer N and the previous-frame-image buffer B in the grid shape of the m×n matrix based on the image division numbers input by the gauger.

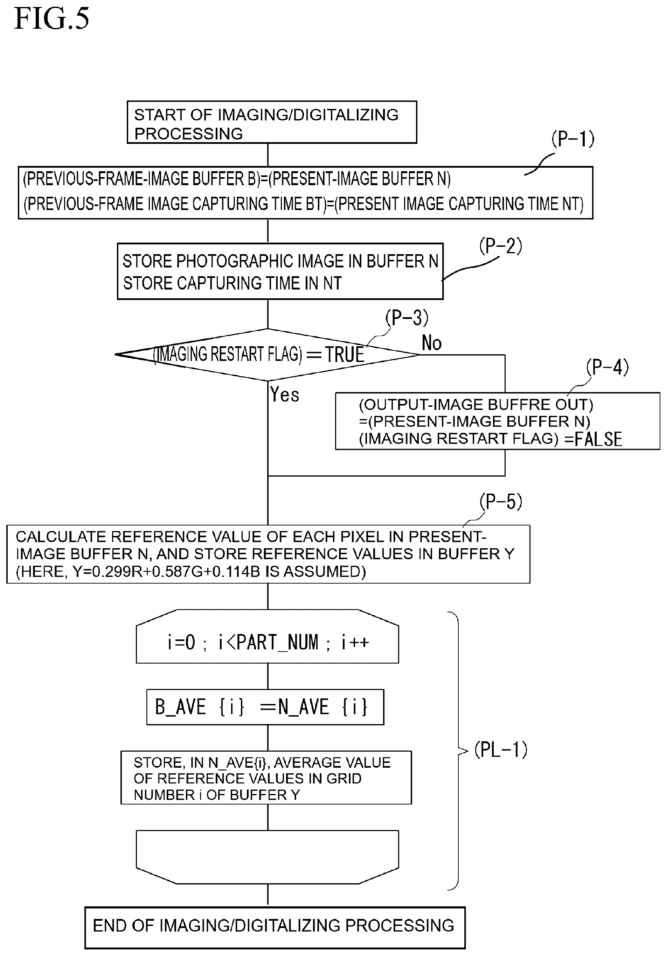

Subsequently, the arithmetic unit 50 executes processing (I-8) of keeping a reference average value N_AVE{PART_NUM} of each of the grids in the present-image buffer N, and a reference average value B_AVE{PART_NUM} of each of the grids (divided areas in the test object eye image) in the previous-frame-image buffer B in the storage means 20. It should be noted that the reference average value N_AVE{PART_NUM} of each of the grids in the present-image buffer N can adopt an average value of numerical values (RGB values, calculation values calculated by substituting the RGB values in a predetermined calculating formula, or the like; see Fig. 5) of the respective pixels in the grid in each of the grids of the present-image buffer N. Similarly, the reference average value B_AVE{PART_NUM} of each of the grids in the previous-frame-image buffer B can adopt an average value of numerical values (RGB values, calculation values calculated by substituting the RGB values in a predetermined calculating formula, or the like; see (P-5) in Fig. 5) of the respective pixels in the grid in each of the grids of the previous-frame-image buffer B.

Subsequently, the arithmetic unit 50 executes processing (I-9) of clearing (zero-clearing) each of the numerical values stored or prepared (kept) in the storage means 20 in steps in the initialization processing I with a preset value (preferably zero) set in advance. In this way, the initialization processing I ends (end of the initialization processing).

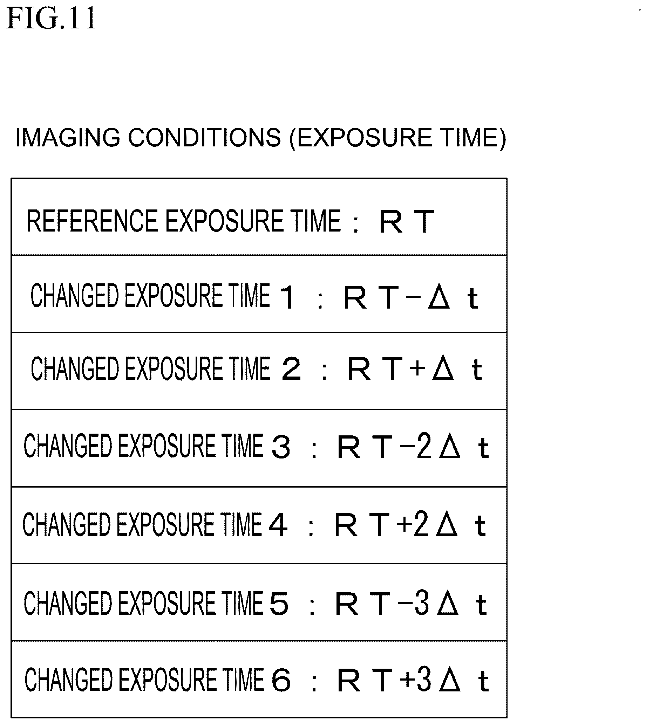

Then, specific processing content in the imaging/digitalizing processing P will be described using Fig. 5. The arithmetic unit 50 executes processing (P-1) of copying the data of the frame image of the test subject eye photographic image in the present-image buffer N to the previous-frame-image buffer B, and copying the present image capturing time NT to the previous-frame image capturing time BT. Subsequently, the arithmetic unit 50 executes processing (P-2) of taking the eye image of the test subject with the imaging means 10 in the reference imaging condition (see Fig. 11) set in the processing (I-5) of setting the reference imaging condition, and then storing the data of the frame image of the test subject eye image thus taken in the present-image buffer N, and storing the current time in the storage means 20 as the present image capturing time NT. As described above, in the present embodiment, the imaging data is constituted by the test subject eye image as image data and capturing time information (capturing time point information) of the test subject eye image, and the test subject eye image and the capturing time information of the test subject eye image are stored in the storage means 20 as separate parameters.

Subsequently, the arithmetic unit 50 confirms (P-3) whether or not the imaging restart flag is TRUE, and when the imaging restart flag is FALSE (No), the arithmetic unit 50 executes processing (P-4) of copying the present-image buffer N to the output-image buffer OUT and setting the imaging restart flag to FALSE. The arithmetic unit 50 executes processing (P-5) of calculating a reference value (a conversion value) obtained by calculating a reference value as the identicalness determining numerical value to be used in an identicalness determination of the images in each of the pixels of the present-image buffer N based on a predetermined calculation value, and then storing the reference value thus calculated in the storage means 20 as a reference value buffer Y. In the present embodiment, the RGB values (an R value, a G value, and a B value) in each of the pixels of the frame image in the present-image buffer N are respectively substituted in the following reference value calculating formula to calculate the reference value related to the luminance. (reference value)=0.299×(R value)+0.587×(G value)+0.114×(B value) It should be noted that the reference value to be stored as the reference value buffer Y is not limited to the reference value related to the luminance in the present embodiment, but may be a reference value obtained directly from at least one of the RGB values (the R value, the G value, and the B value), or by substituting at least one of the RGB values (the R value, the G value, and the B value) in a predetermined conversion formula. On the other hand, when the imaging restart flag is TRUE (Yes), the arithmetic unit 50 directly executes the processing (P-5).

Subsequently, the arithmetic unit 50 repeatedly executes processing of copying the reference average value N_AVE{PART_NUM} of the present image to the reference average value B_AVE{PART_NUM} of the previous-frame image, and processing (PL-1) of calculating an average value of the reference values in all the pixels in the grid in each of the grids (the divided areas in the test subject eye image) of the frame image in the test subject eye image stored in the present-image buffer N using the reference value buffer Y, and then storing the values thus calculated in the storage means 20 as the reference average value N_AVE{PART_NUM} of each of the grids in the present image (the test subject eye image). The arithmetic unit 50 stores the reference average value N_AVE{PART_NUM} in the storage means 20 in all the grids, and then ends (end of the imaging/digitalizing processing) the imaging/digitalizing processing P.

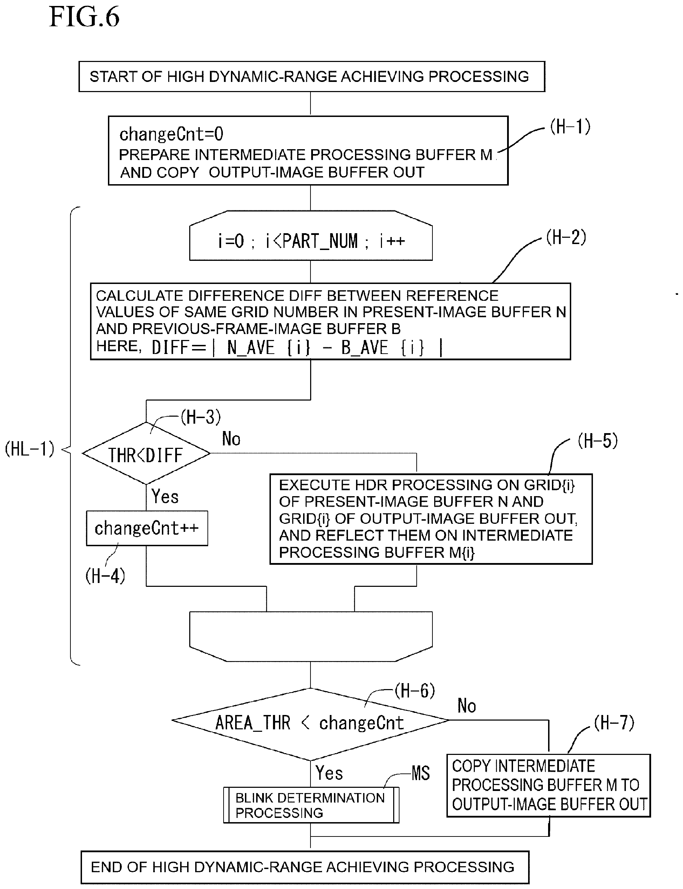

Then, specific processing content in the high dynamic-range achieving processing H will be described using Fig. 6 to Fig. 9. The arithmetic unit 50 executes processing **(H-1)** of setting changeCnt which is a variable for measuring the number of times (the number of dissimilar grids in the frame image) that the grid {i} (i is a serial number of the grid) of the frame image in the present-image buffer N and the grid {i} of the output-image buffer OUT at the same position are dissimilar to each other to 0, and storing changeCnt in the storage means 20, reading out the output-image buffer OUT from the storage means 20, and copying the output-image buffer OUT to an intermediate processing buffer M. Subsequently, the arithmetic unit 50 executes processing (H-2) of calculating an absolute value DIFF of a difference between the reference average value N_AVE{i} in each of the grids of the present-image buffer N and the reference average value B_AVE{i} in each of the grids of the previous-frame-image buffer B.

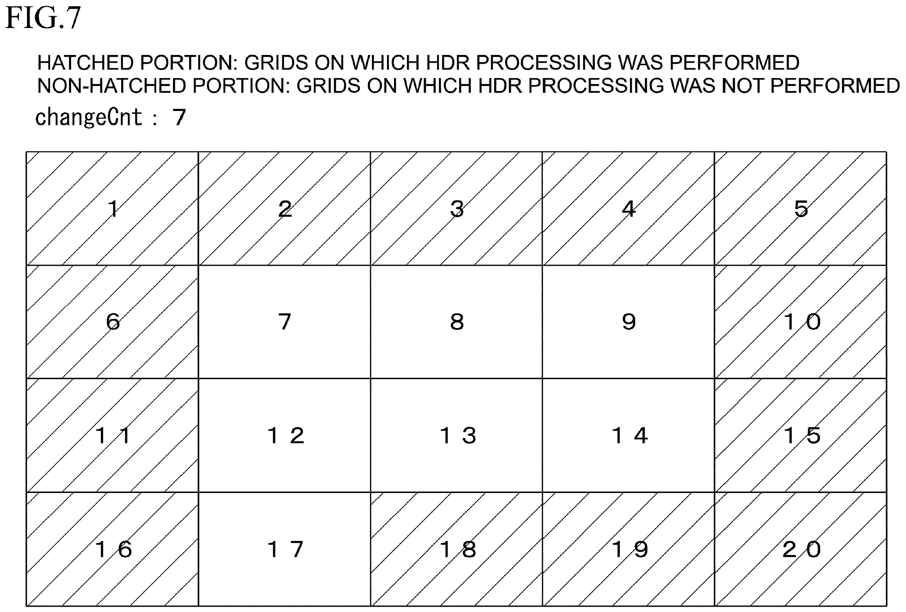

Subsequently, the arithmetic unit 50 compares (H-3) whether or not DIFF thus calculated exceeds the first change amount threshold value THR, and then executes processing (H-4) of adding 1 to changeCnt when DIFF thus calculated exceeds the first change amount threshold value THR. On the other hand, when DIFF thus calculated does not exceed the first change amount threshold value THR (No), the arithmetic unit 50 as the high dynamic-range image generation means executes processing (H-5) of reflecting, on the intermediate processing buffer M, HDR processing data (a hatched portion in Fig. 7) on which high dynamic-range image generation processing is performed using a known method by superimposing the grid {i} of the present-image buffer N and the grid {i} of the output-image buffer OUT (both are the grids at the same position (in the same serial number); the divided areas at the same position in the test subject eye image). The arithmetic unit 50 executes processing (HL-1) of repeating the processing from (H-2) to (H-5) on all the grids.

The arithmetic unit 50 executes the processing (HL-1) on all the grids, and then executes processing (H-6) of confirming whether or not changeCnt exceeds the area change threshold value AREA_THR (corresponding to a second threshold value in the appended claims). It is preferable for the area change threshold value AREA_THR to be a numerical value no smaller than a half of the number of grids (the last number of the serial numbers of the grids). When changeCnt exceeds the area change threshold value AREA_THR (Yes), the arithmetic unit 50 proceeds to a subroutine (MS) of blink determination processing, and then ends (end of the high dynamic-range achieving processing) the high dynamic-range achieving processing H. On the other hand, when changeCnt does not exceed the area change threshold value AREA_THR (No), the arithmetic unit 50 executes processing (H-7) of copying the intermediate processing buffer M to the output-image buffer OUT, and then ends (end of the high dynamic-range achieving processing) the high dynamic-range achieving processing H.

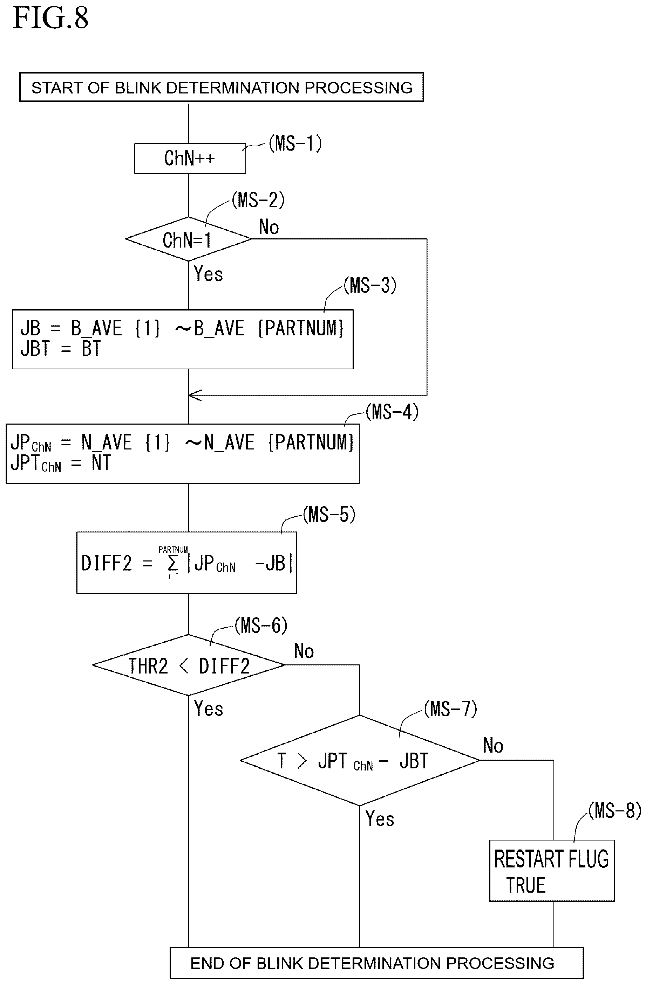

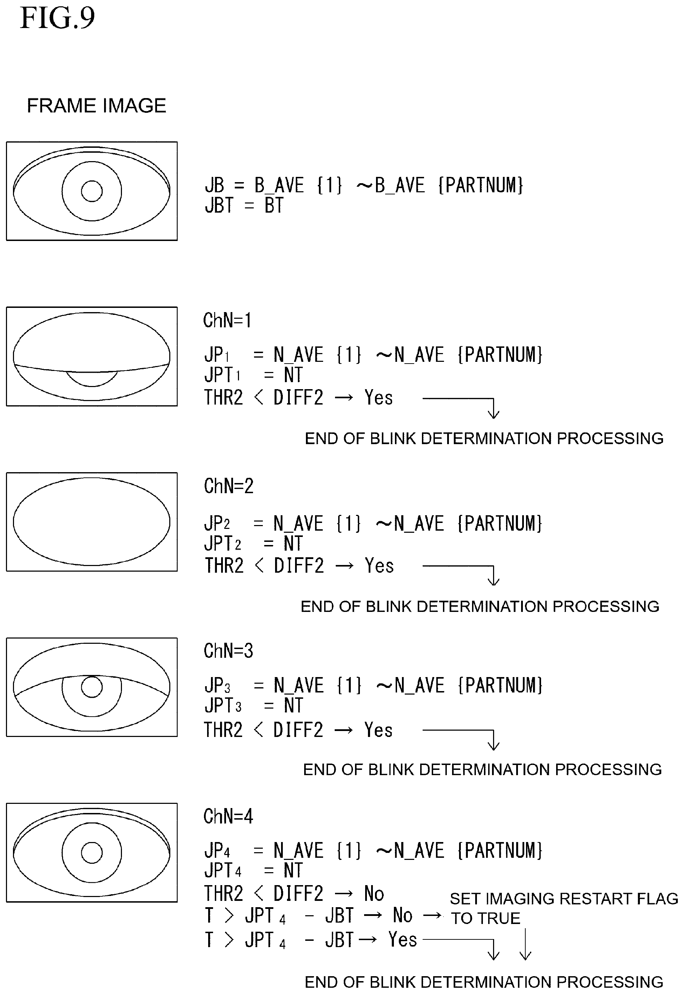

Fig. 8 is a flowchart in the subroutine of the blink determination processing. Fig. 9 is an explanatory diagram showing a specific example in the blink determination processing. Here, the blink determination processing MS when the number of times that it is determined that the present image (the present frame image) has changed in image from the previous frame image in the same imaging condition (the same exposure condition) is four as shown in Fig. 9 will be described. When the blink determination processing MS starts, the arithmetic unit 50 executes processing (MS-1) of adding 1 to a numerical value (ChN=0 at the start of the blink determination processing MS) of ChN which is a variable for counting the number of times of an image change. Subsequently, the arithmetic unit 50 executes processing (MS-2) of confirming whether or not the numerical value of ChN is 1.

When the numerical value of ChN is 1 (Yes in MS-2) at which the test subject eye photographic image changes for the first time, the arithmetic unit 50 executes processing (MS-3) of storing the test subject eye photographic image data of the previous frame to the present image at that time point and the data of the capturing time point of that test subject eye photographic image data in the storage means 20 respectively as the base-image buffer JB and the base image time data JBT. The base-image buffer JB is a reference value in each of the grid in the frame image (a numerical value calculated based on the RGB values in each of the pixels in the grid, and here, a numerical value calculated in substantially the same manner as in Y shown in Fig. 5).

Subsequently, the arithmetic unit 50 executes processing (MS-4) of storing data of the test subject eye photographic image when the test subject eye photographic image changes for the first time and data of the capturing time point of that image data in the storage means 20 respectively as first changed image data JP₁ and first changed image time data JPT₁. On the other hand, when the numerical value of ChN is not 1 (No in MS-2), the arithmetic unit 50 omits the processing (MS-3) of storing the base-image buffer JB and the base image time data JBT in the storage means 20, and executes processing (MS-4) of storing data of the test subject eye photographic image in the ChN-th change and data of the capturing time point of that image data in the storage means 20 respectively as ChN-th changed image data JP_{ChN} and ChN-th changed image time data JPT_{ChN}. It should be noted that a method of calculating the ChN-th changed image data JP_{ChN} can be performed in substantially the same manner as a method of calculating the base-image buffer JB.

Subsequently, the arithmetic unit 50 executes processing (MS-5) of calculating a total difference value DIFF2 of the reference values in the same grid numbers in the ChN-th changed image data JP_{ChN} and the base-image buffer JB. The total difference value DIFF2 is calculated by totalizing absolute values of differences between the reference values in the same grid numbers in the ChN-th changed image data JP_{ChN} and the base-image buffer JB. Subsequently, the arithmetic unit 50 executes processing (MS-6) of determining whether or not the total difference value DIFF2 of the reference values in the same grid numbers in the ChN-th changed image data JP_{ChN} and the base-image buffer JB is greater than the second change amount threshold value THR2 stored in advance in the storage means 20.

When the total difference value DIFF2 of the reference values is greater than the second change amount threshold value THR2 (Yes in MS-6), the blink determination processing MS is ended (end of the blink determination processing). On the other hand, when the total difference value DIFF2 of the reference values is not greater than the second change amount threshold value THR2 (No in MS-6), the arithmetic unit 50 executes processing (MS-7) of determining whether or not a difference between the ChN-th changed image time data JPT_{ChN} and the base image time data JBT is shorter than the blink determination reference time T (corresponding to a first threshold value in the appended claims) stored in advance in the storage means 20.

When the difference between the ChN-th changed image time data JPT_{ChN} and the base image time data JBT is shorter than the blink determination reference time T stored in advance in the storage means 20 (Yes in MS-7), the arithmetic unit 50 ends the blink determination processing MS (end of the blink determination processing). On the other hand, when the difference between the ChN-th changed image time data JPT_{ChN} and the base image time data JBT is longer than the blink determination reference time T stored in advance in the storage means 20 (No in MS-7), the arithmetic unit 50 executes processing (MS-8) of setting the restart flag to TRUE, and then ends the blink determination processing MS (end of the blink determination processing).

The processing contents described hereinabove will more specifically be described with reference to Fig. 9. When it is determined for the first time that the frame image of the test subject eye photographic image at the present time has changed from the frame image of the test subject eye photographic image in the previous frame, the data of the test subject eye photographic image at the present time becomes first changed image data JP₁, and the present image time data becomes first changed image time data JPT₁. Further, the data of the test subject eye photographic image in the previous frame at this time becomes the base-image buffer JB, and the capturing time of the test subject eye photographic image in the previous frame becomes the base image time data JBT. When the total difference value DIFF2 of the reference values in the same grids in the base-image buffer JB and the first changed image data JP₁ exceeds the second change amount threshold value THR2, the arithmetic unit 50 does not perform any processing, and the blink determination processing MS ends. In the case of the pattern shown in Fig. 9, since the total difference value DIFF2 of the reference values in the same grids exceeds the second change amount threshold value THR2 in all of the first changed image data JP₁ to the third changed image data JP₃, the arithmetic unit 50 does not perform any processing, and the blink determination processing MS ends.

Further, since the total difference value DIFF2 of the reference values in the same grid in the base-image buffer JB and the fourth changed image data JP₄ does not exceed the second change amount threshold value THR2 regarding the fourth changed image data JP₄, the arithmetic unit 50 determines as a result that the fourth changed image data JP₄ is the same image as that in the base-image buffer JB. In this case, the arithmetic unit 50 calculates an amount of change with time from the base-image buffer JB to the fourth changed image data JP₄ which is the same image as that in the base-image buffer JB using a difference between the fourth changed image time data JPT₄ and the base image time data JBT, and when the amount of change with time thus calculated is within a range of the blink determination reference time T, the arithmetic unit 50 does not perform any processing, and the blink determination processing MS ends. On the other hand, when the amount of change with time thus calculated exceeds the range of the blink determination reference time T, the arithmetic unit 50 determines that the time from when the test subject eye photographic image changes from the base-image buffer JB to when the test subject eye photographic image is restored again to the test subject eye photographic image (here, the fourth changed image data JP₄) which is the same as that in the base-image buffer JB exceeds the blink determination reference time T, and therefore, in order to discard the past data, the arithmetic unit 50 executes processing (MS-8) of setting the imaging restart flag to TRUE, and then ends the blink determination processing MS.

When the subroutine of the blink determination processing MS inserted into the high dynamic-range achieving processing H ends, the high dynamic-range achieving processing H also ends (end of the high dynamic-range achieving processing).

By including the blink determination processing MS in the processing of generating a high dynamic-range image as described above, even when an image when blinking is included in the photographic images in the same imaging condition (the same exposure condition), no disturbance occurs in the high dynamic-range image unless only the test subject eye photographic image when blinking is used in the processing of generating the high dynamic-range image. Further, since it is possible to use the test subject eye photographic image before blinking in the processing of generating the high dynamic-range image, the discard of the data of the test subject eye photographic image can be made as little as possible, and the processing of generating the high dynamic-range image in a short time becomes possible.

In contrast, when the time from when the image data changes from that in the base-image buffer JB to when the image data is restored again to the test subject eye photographic image which is the same as that in the base-image buffer JB exceeds the blink determination reference time T, the test subject eye photographic image is numerically restored to the base image data, but there is a high possibility that no blink occurs, and there is a possibility that a change occurs in a sight of the test subject in the test subject eye image. Since there is a possibility that a disturbance occurs in the high dynamic-range image when performing the processing of generating the high dynamic-range image by combining the test subject eye photographic image data in which the sight of the test subject changes with the test subject eye photographic image before the sight changes, it is preferable to perform the processing of generating the high dynamic-range image using the test subject eye photographic image data which is obtained by performing imaging once again without using such test subject eye photographic image data.

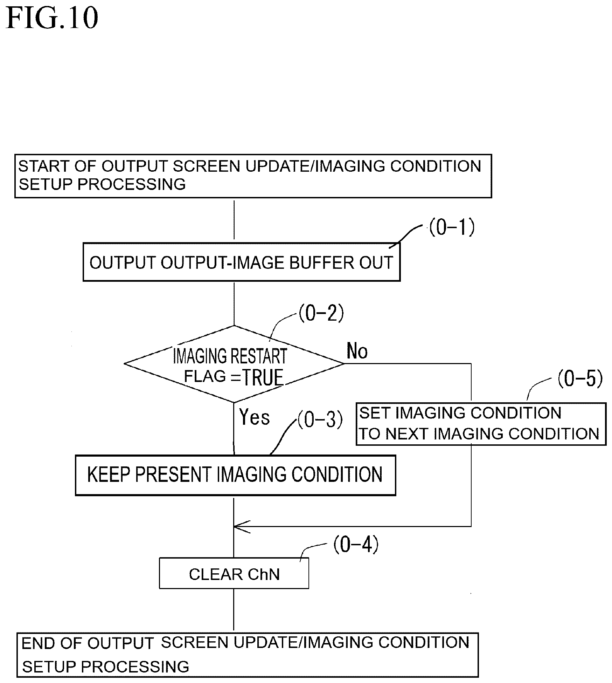

Then, the arithmetic unit 50 executes the output screen update/imaging condition setup processing O shown in Fig. 10. When the output screen update/imaging condition setup processing O starts, the arithmetic unit 50 as an image display control means executes processing (O-1) of outputting data stored in the output-image buffer OUT to a display unit such as a display. Subsequently, the arithmetic unit 50 executes processing (O-2) of determining whether or not the imaging restart flag is TRUE. When the imaging restart flag is TRUE (Yes in O-2), the arithmetic unit 50 executes processing (O-3) of keeping the imaging condition in the imaging means 10 in the present imaging condition, and subsequently executes processing (O-4) of clearing ChN which is a variable of the number of times of the image change, and then ends the output screen update/imaging condition setup processing O (end of the output screen update/imaging condition setup processing). On the other hand, when the imaging restart flag is not TRUE (No in O-2), the arithmetic unit 50 executes processing (O-5) of setting the imaging condition of the imaging means 10 to a next imaging condition, and subsequently executes the processing (O-4) of clearing ChN which is the variable of the number of times of the image change, and then ends the output screen update/imaging condition setup processing O (end of the output screen update/imaging condition setup processing).

It should be noted that as the imaging condition of the imaging means 10 in the present embodiment, a plurality of imaging conditions is set based on the exposure time as shown in Fig. 11. Specifically, assuming the imaging condition in which the exposure time is a reference exposure time RT as a reference imaging condition, a changed exposure time 1 (RT-Δt) obtained by shortening the exposure time by a predetermined time Δt with respect to the reference exposure time RT, a changed exposure time 2 (RT+Δt) obtained by elongating the exposure time by the predetermined time Δt with respect to the reference exposure time RT, a changed exposure time 3 (RT-2Δt) obtained by shortening the exposure time by a time twice as much as the predetermined time Δt with respect to the reference exposure time RT, a changed exposure time 4 (RT+2Δt) obtained by elongating the exposure time by a time twice as much as the predetermined time Δt with respect to the reference exposure time RT, a changed exposure time 5 (RT-3Δt) obtained by shortening the exposure time by a time three times as much as the predetermined time Δt with respect to the reference exposure time RT, and a changed exposure time 6 (RT+3Δt) obtained by elongating the exposure time by a time three times as much as the predetermined time Δt with respect to the reference exposure time RT are set. As described above, the imaging conditions of the imaging means 10 in the present embodiment are set using integer multiples of the predetermined time Δt set in advance as a variation width with reference to the reference exposure time RT, but this configuration is not a limitation. Further, the change in the imaging condition may be set based on other elements than the exposure time.

As described hereinabove, according to the present invention, the disturbance in the image on which the high dynamic-range achieving processing is performed can favorably be avoided by stopping the execution of the high dynamic-range achieving processing H on the frame image during the period in which the test subject eye photographic image data is different from the base image, and resuming the high dynamic-range achieving processing H from the time point at which the test subject eye photographic image data is restored to the base image as long as the elapsed time until the test subject eye photographic image data is restored again to the base image data is within a predetermined time when it is determined that the test subject eye photographic image data is different from the base image when performing the processing of making the test subject eye photographic image data into the high dynamic-range image. By adopting such high dynamic-range achieving processing H, it is possible to reduce the discard (discard of the test subject eye photographic image data which is not used in the high dynamic-range achieving processing H) of the test subject eye photographic image data, and it favorably becomes possible to efficiently perform the high dynamic-range achieving processing H.

Further, a configuration in which the storage means 20, the image display unit 30, and the arithmetic unit 50 are integrated with the ophthalmic apparatus 100 is illustrated in the embodiment described above, but the present invention is not limited to this configuration. It is possible to adopt a configuration in which at least one of the storage means 20, the image display unit 30, and the arithmetic unit 50 is coupled via the external input output unit 40. As the arithmetic unit 50 coupled via the external input output unit 40, a personal computer in which a high dynamic-range image generation program according to the present invention is installed in an executable manner can be exemplified.

Although the ophthalmic apparatus 100 in the present embodiment is hereinabove described, the present invention is not limited to the embodiment described above. For example, in the embodiment described above, it is possible to adopt a configuration in which a base image selecting reference image is stored in advance in the storage means 20, the arithmetic unit 50 makes the image display unit 30 display the base image selecting reference image, and the gauger determines similarity between the test subject eye photographic image data and the base image selecting reference image to select the base image besides the configuration in which the gauger sets, as the base image, the test subject eye photographic image data selected from the test subject eye photographic image data when setting the base image. Further, it is also possible to adopt a configuration in which the arithmetic unit 50 performs similarity determination processing between images with a known method or the image identicalness determination means described hereinabove, performs comparison of the similarity between the base image selecting reference image and the photographic image, and the arithmetic unit 50 sets the photographic image having the highest similarity as the base image.

Further, the ophthalmic apparatus 100 having the arithmetic unit 50 which functions as the high dynamic-range image generation means and the method for generating the high dynamic-range image are described in the embodiment described above, but the embodiment described above is not a limitation. There is also a program for generating the high dynamic-range image which can make the arithmetic unit 50 of the ophthalmic apparatus 100 execute the method of generating the high dynamic-range image described in the above embodiment.

Further, a moving image at 30 to 60 frames per second is illustrated as the test subject eye photographic image in the embodiment described above, but the test subject eye photographic image is not limited to this configuration. A moving image which exceeds 60 frames per second may be adopted, and it is possible to adopt the test subject eye photographic image in which a plurality of still images and the capturing time data of the respective still images are paired respectively.

Further, the configuration of performing the processing of making the image display unit 30 display both the base image and the high dynamic-range image stored in the output-image buffer OUT is illustrated as the arithmetic unit 50 as the image display control means in the embodiment described hereinabove, but the arithmetic unit 50 is not limited to this configuration. It is sufficient for the arithmetic unit 50 to make the image display unit 30 display at least one of the base image and the high dynamic-range image stored in the output-image buffer OUT. In this case, it is sufficient to make the image display unit 30 display the base image until the processing of generating the high dynamic-range image is completed, and make the image display unit 30 display the high dynamic-range image stored in the output-image buffer OUT after the processing of generating the high dynamic-range image is completed.

Further, it is possible to adopt a configuration in which the embodiment and the modified example described hereinabove are appropriately combined with each other.

Other features of the present invention are set out in the following numbered clauses.
[Clause 1] An ophthalmic apparatus (100) comprising:
   an imaging means (10) configured to take test subject eye images at constant time intervals for each of exposure conditions different from each other;
   a storage means (20) configured to store, as imaging data, the test subject eye image together with capturing time information, and store, as a base image, the test subject eye image selected by similarly to a base image selecting reference image or a gauger out of the test subject eye images in the imaging data;
   an output image initialization means (50) configured to store a copy of the base image in the storage means as an output image;
   a high dynamic-range image generation means (50) configured to perform high dynamic-range achieving processing on the output image to generate a high dynamic-range image; and
   an image identicalness determination means (50) configured to determine whether or not the test subject eye image in the imaging data is same as the base image, wherein
   the high dynamic-range image generation means (50) executes processing of updating the output image by superimposing the test subject eye image on the output image and storing the output image in the storage means (20) on the test subject eye image in same exposure condition to generate the high dynamic-range image only if the image identicalness determination means (50) compares similarity between the test subject eye image and the base image in a chronological order of the capturing time information, and determines that the test subject eye image and the base image are same as each other.
[Clause 2] The ophthalmic apparatus (100) according to Clause **1,** wherein
   the high dynamic-range image generation means (50)
   executes processing of continuously using the output image if a difference between the capturing time information in a case in which the image identicalness determination means (50) first determines that the test subject eye image is different from the base image and the capturing time information in a case in which the image identicalness determination means determines that the test subject eye image is restored to the base image is within a first threshold value set in advance, and
   executes processing of making the imaging means take the test subject eye image once again, processing of storing, as the imaging data, the test subject eye image taken once again together with the capturing time information in the storage means (20), processing of storing, as the base image, the test subject eye image selected from the test subject eye images in the imaging data in the storage means, and processing of making the output image initialization means (50) store a copy of the base image in the storage means (20) as the output image if the difference between the capturing time information in a case in which the image identicalness determination means (50) first determines that the test subject eye image is different from the base image and the capturing time information in a case in which the image identicalness determination means determines that the test subject eye image is restored to the base image exceeds the first threshold value set in advance.
[Clause 3] The ophthalmic apparatus (100) according to Clause 1 or 2, wherein
   the image identicalness determination means (50) executes
   processing of dividing each of the base image, the test subject eye image, and the output image into a plurality of divided areas same in shape and arrangement,
   processing of updating the output image by superimposing the divided areas in the test subject eye image on the divided areas in the output image if a difference in identicalness determining numerical value between the divided areas is no greater than a second threshold value set in advance in each of the divided areas of the test subject eye image and the base image at positions same as each other, and
   processing of keeping the output image if the difference in identicalness determining numerical value between the divided areas exceeds the second threshold value in each of the divided areas of the test subject eye image and the base image at positions same as each other.
[Clause 4] The ophthalmic apparatus (100) according to Clause 3, wherein
   based on either one of at least one of values (an R value, a G value, and a B value) out of RGB values in each of pixels of the test subject eye image and the base image, or a conversion value obtained by converting the RGB values with a conversion formula set in advance, the identicalness determining numerical value is an average value of the values or the conversion values of the pixels in each of the divided areas.
[Clause 5] The ophthalmic apparatus (100) according to Clause 1 or 2, further comprising:
   an image display unit (30) configured to display at least one of the base image and the output image; and
   an image display control means configured to make the image display unit (30) display at least one of the base image and the output image, wherein
   the image display control means displays the output image on the image display unit (30) every time the output image is updated.
[Clause 6] The ophthalmic apparatus (100) according to Clause 3, further comprising:
   an image display unit (30) configured to display at least one of the base image and the output image; and
   an image display control means configured to make the image display unit (30) display at least one of the base image and the output image, wherein
   the image display control means displays the output image on the image display unit (30) every time the output image is updated.
[Clause 7] The ophthalmic apparatus (100) according to Clause 4, further comprising:
   an image display unit (30) configured to display at least one of the base image and the output image; and
   an image display control means configured to make the image display unit (30) display at least one of the base image and the output image, wherein
   the image display control means displays the output image on the image display unit (30) every time the output image is updated.
[Clause 8] A method for generating a high dynamic-range image for an ophthalmic apparatus (100) used for the ophthalmic apparatus equipped with an imaging means (10) configured to take an eye image of a test subject, a storage means (20), and an arithmetic unit (50), the method comprising:
   a step of making, by the arithmetic unit (50), the imaging means (10) take test subject eye images at constant time intervals for each of exposure conditions different from each other;
   a step of making, by the arithmetic unit (50), the storage means (20) store, as imaging data, the test subject eye image together with capturing time information;
   a step of making, by the arithmetic unit (50), the storage means (20) store, as a base image, the test subject eye image which is selected based on similarity to a base image selecting reference image, or selected by a gauger, out of the test subject eye images in the imaging data;
   a step of making, by the arithmetic unit (50), the storage means (20) store a copy of the base image as an output image;
   a step of performing, by the arithmetic unit (50), high dynamic-range achieving processing on the output image to generate a high dynamic-range image; and
   a step of determining, by the arithmetic unit (50), whether or not the test subject eye image in the imaging data is same as the base image, wherein
   the step of generating the high dynamic-range image includes a step of making the arithmetic unit (50) compare similarity between the test subject eye image and the base image in a chronological order of the capturing time information, and then making the arithmetic unit (50) update the output image by superimposing the test subject eye image on the output image and store the output image in the storage means (20) only when it is determined that the test subject eye image and the base image are same as each other, and a step of comparing, by the arithmetic unit (50), the similarity between the test subject eye image and the base image in a chronological order of the capturing time information, and then making the arithmetic unit (50) keep the output image when it is determined that the test subject eye image is different from the base image.
[Clause 9] The method for generating a high dynamic-range image for an ophthalmic apparatus according to Clause 8, further comprising:
   a step of continuously using the output image when a difference between the capturing time information when the arithmetic unit (50) first determines that the test subject eye image is different from the base image and the capturing time information when the arithmetic unit (50) determines that the test subject eye image is restored to the base image is within a first threshold value set in advance, and
   when the difference between the capturing time information when the arithmetic unit (50) first determines that the test subject eye image is different from the base image and the capturing time information when the arithmetic unit (50) determines that the test subject eye image is restored to the base image exceeds the first threshold value set in advance, a step of making the imaging means (10) take the test subject eye image once again, a step of storing, as the imaging data, the test subject eye image taken once again together with the capturing time information in the storage means (20), a step of storing, as the base image, the test subject eye image selected from the test subject eye images in the imaging data in the storage means (20), and a step of making the arithmetic unit (50) store a copy of the base image in the storage means (20) as the output image.
[Clause 10] The method for generating a high dynamic-range image for an ophthalmic apparatus according to Clause 8 or 9, wherein
   a step of dividing, by the arithmetic unit (50), each of the base image, the test subject eye image, and the output image into a plurality of divided areas same in shape and arrangement,
   a step of updating the output image by superimposing the divided areas in the test subject eye image on the divided areas in the output image when the arithmetic unit (50) determines that a difference in identicalness determining numerical value between the divided areas is no greater than a second threshold value set in advance in each of the divided areas of the test subject eye image and the base image at positions same as each other, and
   a step of keeping the output image when the arithmetic unit (50) determines that the difference in identicalness determining numerical value between the divided areas exceeds the second threshold value in each of the divided areas of the test subject eye image and the base image at positions same as each other are executed.
[Clause 11] The method for generating a high dynamic-range image for an ophthalmic apparatus according to Clause 10, wherein
   based on either one of at least one of values (an R value, a G value, and a B value) out of RGB values in each of pixels of the test subject eye image and the base image, or a conversion value obtained by converting the RGB values with a conversion formula set in advance, the arithmetic unit (50) uses an average value of the values or the conversion values of the pixels in each of the divided areas as the identicalness determining numerical value.
[Clause 12] The method for generating a high dynamic-range image for an ophthalmic apparatus according to Clause 8 or 9, wherein
   the ophthalmic apparatus (100) further includes an image display unit (30) configured to display at least one of the base image and the output image, and an image display control means configured to make the image display unit (30) display at least one of the base image and the output image, and
   the method further comprising a step of making, by the arithmetic unit (50), the image display unit (30) display the output image every time the output image is updated.
[Clause 13] The method for generating a high dynamic-range image for an ophthalmic apparatus according to Clause 10, wherein
   the ophthalmic apparatus (100) further includes an image display unit (30) configured to display at least one of the base image and the output image, and an image display control means configured to make the image display unit (30) display at least one of the base image and the output image, and
   the method further comprising a step of displaying, by the arithmetic unit (50), the output image on the image display unit (30) every time the output image is updated.
[Clause 14] The method for generating a high dynamic-range image for an ophthalmic apparatus according to Clause 11, wherein
   the ophthalmic apparatus (100) further includes an image display unit (30) configured to display at least one of the base image and the output image, and an image display control means configured to make the image display unit (30) display at least one of the base image and the output image, and
   the method further comprising a step of displaying, by the arithmetic unit (50), the output image on the image display unit (30) every time the output image is updated.
[Clause 15] A program for generating a high dynamic-range image for an ophthalmic apparatus (100) used for the ophthalmic apparatus equipped with an imaging means (10) configured to take an eye image of a test subject, a storage means (20), and an arithmetic unit (50), the program making the arithmetic unit (50) execute:
   processing of making the imaging means (10) take test subject eye images at constant time intervals for each of exposure conditions different from each other;
   processing of making the storage means (20) store, as imaging data, the test subject eye image together with capturing time information;
   processing of making the storage means (20) store, as a base image, the test subject eye image which is selected based on similarity to a base image selecting reference image, or selected by a gauger, out of the test subject eye images in the imaging data;
   processing of making the storage means (20) store a copy of the base image as an output image;
   processing of generating a high dynamic-range image by performing high dynamic-range achieving processing on the output image; and
   processing of determining whether or not the test subject eye image in the imaging data is same as the base image, wherein
   the processing of generating the high dynamic-range image includes
   processing of comparing similarity between the test subject eye image and the base image in a chronological order of the capturing time information, and then updating the output image by superimposing the test subject eye image on the output image and storing the output image in the storage means (20) when it is determined that the test subject eye image and the base image are same as each other, and
   processing of comparing the similarity between the test subject eye image and the base image in a chronological order of the capturing time information, and then keeping the output image when it is determined that the test subject eye image is different from the base image.
[Clause 16] The program for generating a high dynamic-range image for an ophthalmic apparatus according to Clause 15, wherein
   the arithmetic unit (50) is made to execute
   processing of continuously using the output image when a difference between the capturing time information when it is first determined that the test subject eye image is different from the base image and the capturing time information when it is determined that the test subject eye image is restored to the base image is within a first threshold value set in advance, and
   when the difference between the capturing time information when it is first determined that the test subject eye image is different from the base image and the capturing time information when it is determined that the test subject eye image is restored to the base image exceeds the first threshold value set in advance, processing of making the imaging means take the test subject eye image once again, processing of storing, as the imaging data, the test subject eye image taken once again together with the capturing time information in the storage means (20), processing of storing, as the base image, the test subject eye image selected from the test subject eye images in the imaging data in the storage means (20), and processing of storing a copy of the base image in the storage means (20) as the output image.
[Clause 17] The program for generating a high dynamic-range image for an ophthalmic apparatus according to Clause 15 or 16, wherein
   the arithmetic unit (50) is made to execute
   processing of dividing each of the base image, the test subject eye image, and the output image into a plurality of divided areas same in shape and arrangement,
   processing of updating the output image by superimposing the divided areas in the test subject eye image on the divided areas in the output image when it is determined that a difference in identicalness determining numerical value between the divided areas is no greater than a second threshold value set in advance in each of the divided areas of the test subject eye image and the base image at positions same as each other, and
   processing of keeping the output image when it is determined that the difference in identicalness determining numerical value between the divided areas exceeds the second threshold value in each of the divided areas of the test subject eye image and the base image at positions same as each other.
[Clause 18] The program for generating a high dynamic-range image for an ophthalmic apparatus according to Clause 17, wherein
   based on either one of at least one of values (an R value, a G value, and a B value) out of RGB values in each of pixels of the test subject eye image and the base image, or a conversion value obtained by converting the RGB values with a conversion formula set in advance, the arithmetic unit (50) is made to use an average value of the values or the conversion values of the pixels in each of the divided areas as the identicalness determining numerical value.
[Clause 19] The program for generating a high dynamic-range image for an ophthalmic apparatus according to Clause 15 or 16, wherein
   the ophthalmic apparatus (100) further includes an image display unit (30) configured to display at least one of the base image and the output image, and an image display control means configured to make the image display unit (30) display at least one of the base image and the output image, and
   the arithmetic unit (50) is made to display the output image on the image display unit (30) every time the output image is updated.
[Clause 20] The program for generating a high dynamic-range image for an ophthalmic apparatus according to Clause 17, wherein
   the ophthalmic apparatus (100) further includes an image display unit (30) configured to display at least one of the base image and the output image, and an image display control means configured to make the image display unit (30) display at least one of the base image and the output image, and
   the arithmetic unit (50) is made to display the output image on the image display unit (30) every time the output image is updated.
[Clause 21] The program for generating a high dynamic-range image for an ophthalmic apparatus according to Clause 18, wherein
   the ophthalmic apparatus (100) further includes an image display unit (30) configured to display at least one of the base image and the output image, and an image display control means configured to make the image display unit (30) display at least one of the base image and the output image, and
   the arithmetic unit (50) is made to display the output image on the image display unit (30) every time the output image is updated.

## Claims

1. An ophthalmic apparatus (100) comprising:
an imaging means (10) configured to take test subject eye images at constant time intervals for each of exposure conditions different from each other;
a storage means (20) configured to store, as imaging data, the test subject eye image together with capturing time information, and store, as a base image, the test subject eye image selected by similarly to a base image selecting reference image or a gauger out of the test subject eye images in the imaging data;
an output image initialization means (50) configured to store a copy of the base image in the storage means as an output image;
a high dynamic-range image generation means (50) configured to perform high dynamic-range achieving processing on the output image to generate a high dynamic-range image; and
an image identicalness determination means (50) configured to determine whether or not the test subject eye image in the imaging data is same as the base image, wherein
the high dynamic-range image generation means (50) executes processing of updating the output image by superimposing the test subject eye image on the output image and storing the output image in the storage means (20) on the test subject eye image in same exposure condition to generate the high dynamic-range image only if the image identicalness determination means (50) compares similarity between the test subject eye image and the base image in a chronological order of the capturing time information, and determines that the test subject eye image and the base image are same as each other.

2. The ophthalmic apparatus (100) according to claim **1,** wherein
the high dynamic-range image generation means (50)
executes processing of continuously using the output image if a difference between the capturing time information in a case in which the image identicalness determination means (50) first determines that the test subject eye image is different from the base image and the capturing time information in a case in which the image identicalness determination means determines that the test subject eye image is restored to the base image is within a first threshold value set in advance, and
executes processing of making the imaging means take the test subject eye image once again, processing of storing, as the imaging data, the test subject eye image taken once again together with the capturing time information in the storage means (20), processing of storing, as the base image, the test subject eye image selected from the test subject eye images in the imaging data in the storage means, and processing of making the output image initialization means (50) store a copy of the base image in the storage means (20) as the output image if the difference between the capturing time information in a case in which the image identicalness determination means (50) first determines that the test subject eye image is different from the base image and the capturing time information in a case in which the image identicalness determination means determines that the test subject eye image is restored to the base image exceeds the first threshold value set in advance.

3. The ophthalmic apparatus (100) according to claim 1 or 2, wherein
the image identicalness determination means (50) executes
processing of dividing each of the base image, the test subject eye image, and the output image into a plurality of divided areas same in shape and arrangement,
processing of updating the output image by superimposing the divided areas in the test subject eye image on the divided areas in the output image if a difference in identicalness determining numerical value between the divided areas is no greater than a second threshold value set in advance in each of the divided areas of the test subject eye image and the base image at positions same as each other, and
processing of keeping the output image if the difference in identicalness determining numerical value between the divided areas exceeds the second threshold value in each of the divided areas of the test subject eye image and the base image at positions same as each other,
optionally wherein the ophthalmic apparatus (100) further comprises:
an image display unit (30) configured to display at least one of the base image and the output image; and
an image display control means configured to make the image display unit (30) display at least one of the base image and the output image, wherein
the image display control means displays the output image on the image display unit (30) every time the output image is updated.

4. The ophthalmic apparatus (100) according to claim 3, wherein
based on either one of at least one of values (an R value, a G value, and a B value) out of RGB values in each of pixels of the test subject eye image and the base image, or a conversion value obtained by converting the RGB values with a conversion formula set in advance, the identicalness determining numerical value is an average value of the values or the conversion values of the pixels in each of the divided areas.

5. The ophthalmic apparatus (100) according to claim 1 or 2, further comprising:
an image display unit (30) configured to display at least one of the base image and the output image; and
an image display control means configured to make the image display unit (30) display at least one of the base image and the output image, wherein
the image display control means displays the output image on the image display unit (30) every time the output image is updated.

6. A method for generating a high dynamic-range image for an ophthalmic apparatus (100) used for the ophthalmic apparatus equipped with an imaging means (10) configured to take an eye image of a test subject, a storage means (20), and an arithmetic unit (50), the method comprising:
a step of making, by the arithmetic unit (50), the imaging means (10) take test subject eye images at constant time intervals for each of exposure conditions different from each other;
a step of making, by the arithmetic unit (50), the storage means (20) store, as imaging data, the test subject eye image together with capturing time information;
a step of making, by the arithmetic unit (50), the storage means (20) store, as a base image, the test subject eye image which is selected based on similarity to a base image selecting reference image, or selected by a gauger, out of the test subject eye images in the imaging data;
a step of making, by the arithmetic unit (50), the storage means (20) store a copy of the base image as an output image;
a step of performing, by the arithmetic unit (50), high dynamic-range achieving processing on the output image to generate a high dynamic-range image; and
a step of determining, by the arithmetic unit (50), whether or not the test subject eye image in the imaging data is same as the base image, wherein
the step of generating the high dynamic-range image includes a step of making the arithmetic unit (50) compare similarity between the test subject eye image and the base image in a chronological order of the capturing time information, and then making the arithmetic unit (50) update the output image by superimposing the test subject eye image on the output image and store the output image in the storage means (20) only when it is determined that the test subject eye image and the base image are same as each other, and a step of comparing, by the arithmetic unit (50), the similarity between the test subject eye image and the base image in a chronological order of the capturing time information, and then making the arithmetic unit (50) keep the output image when it is determined that the test subject eye image is different from the base image.

7. The method for generating a high dynamic-range image for an ophthalmic apparatus according to claim 6, further comprising:
a step of continuously using the output image when a difference between the capturing time information when the arithmetic unit (50) first determines that the test subject eye image is different from the base image and the capturing time information when the arithmetic unit (50) determines that the test subject eye image is restored to the base image is within a first threshold value set in advance, and
when the difference between the capturing time information when the arithmetic unit (50) first determines that the test subject eye image is different from the base image and the capturing time information when the arithmetic unit (50) determines that the test subject eye image is restored to the base image exceeds the first threshold value set in advance, a step of making the imaging means (10) take the test subject eye image once again, a step of storing, as the imaging data, the test subject eye image taken once again together with the capturing time information in the storage means (20), a step of storing, as the base image, the test subject eye image selected from the test subject eye images in the imaging data in the storage means (20), and a step of making the arithmetic unit (50) store a copy of the base image in the storage means (20) as the output image.

8. The method for generating a high dynamic-range image for an ophthalmic apparatus according to claim 6 or 7, wherein
a step of dividing, by the arithmetic unit (50), each of the base image, the test subject eye image, and the output image into a plurality of divided areas same in shape and arrangement,
a step of updating the output image by superimposing the divided areas in the test subject eye image on the divided areas in the output image when the arithmetic unit (50) determines that a difference in identicalness determining numerical value between the divided areas is no greater than a second threshold value set in advance in each of the divided areas of the test subject eye image and the base image at positions same as each other, and
a step of keeping the output image when the arithmetic unit (50) determines that the difference in identicalness determining numerical value between the divided areas exceeds the second threshold value in each of the divided areas of the test subject eye image and the base image at positions same as each other are executed,
optionally wherein
the ophthalmic apparatus (100) further includes an image display unit (30) configured to display at least one of the base image and the output image, and an image display control means configured to make the image display unit (30) display at least one of the base image and the output image, and
the method further comprising a step of displaying, by the arithmetic unit (50), the output image on the image display unit (30) every time the output image is updated.

9. The method for generating a high dynamic-range image for an ophthalmic apparatus according to claim 8, wherein
based on either one of at least one of values (an R value, a G value, and a B value) out of RGB values in each of pixels of the test subject eye image and the base image, or a conversion value obtained by converting the RGB values with a conversion formula set in advance, the arithmetic unit (50) uses an average value of the values or the conversion values of the pixels in each of the divided areas as the identicalness determining numerical value.

10. The method for generating a high dynamic-range image for an ophthalmic apparatus according to claim 6 or 7, wherein
the ophthalmic apparatus (100) further includes an image display unit (30) configured to display at least one of the base image and the output image, and an image display control means configured to make the image display unit (30) display at least one of the base image and the output image, and
the method further comprising a step of making, by the arithmetic unit (50), the image display unit (30) display the output image every time the output image is updated.

11. A program for generating a high dynamic-range image for an ophthalmic apparatus (100) used for the ophthalmic apparatus equipped with an imaging means (10) configured to take an eye image of a test subject, a storage means (20), and an arithmetic unit (50), the program making the arithmetic unit (50) execute:
processing of making the imaging means (10) take test subject eye images at constant time intervals for each of exposure conditions different from each other;
processing of making the storage means (20) store, as imaging data, the test subject eye image together with capturing time information;
processing of making the storage means (20) store, as a base image, the test subject eye image which is selected based on similarity to a base image selecting reference image, or selected by a gauger, out of the test subject eye images in the imaging data;
processing of making the storage means (20) store a copy of the base image as an output image;
processing of generating a high dynamic-range image by performing high dynamic-range achieving processing on the output image; and
processing of determining whether or not the test subject eye image in the imaging data is same as the base image, wherein
the processing of generating the high dynamic-range image includes
processing of comparing similarity between the test subject eye image and the base image in a chronological order of the capturing time information, and then updating the output image by superimposing the test subject eye image on the output image and storing the output image in the storage means (20) when it is determined that the test subject eye image and the base image are same as each other, and
processing of comparing the similarity between the test subject eye image and the base image in a chronological order of the capturing time information, and then keeping the output image when it is determined that the test subject eye image is different from the base image.

12. The program for generating a high dynamic-range image for an ophthalmic apparatus according to claim **11,** wherein
the arithmetic unit (50) is made to execute
processing of continuously using the output image when a difference between the capturing time information when it is first determined that the test subject eye image is different from the base image and the capturing time information when it is determined that the test subject eye image is restored to the base image is within a first threshold value set in advance, and
when the difference between the capturing time information when it is first determined that the test subject eye image is different from the base image and the capturing time information when it is determined that the test subject eye image is restored to the base image exceeds the first threshold value set in advance, processing of making the imaging means take the test subject eye image once again, processing of storing, as the imaging data, the test subject eye image taken once again together with the capturing time information in the storage means (20), processing of storing, as the base image, the test subject eye image selected from the test subject eye images in the imaging data in the storage means (20), and processing of storing a copy of the base image in the storage means (20) as the output image.

13. The program for generating a high dynamic-range image for an ophthalmic apparatus according to claim **11** or 12, wherein
the arithmetic unit (50) is made to execute
processing of dividing each of the base image, the test subject eye image, and the output image into a plurality of divided areas same in shape and arrangement,
processing of updating the output image by superimposing the divided areas in the test subject eye image on the divided areas in the output image when it is determined that a difference in identicalness determining numerical value between the divided areas is no greater than a second threshold value set in advance in each of the divided areas of the test subject eye image and the base image at positions same as each other, and
processing of keeping the output image when it is determined that the difference in identicalness determining numerical value between the divided areas exceeds the second threshold value in each of the divided areas of the test subject eye image and the base image at positions same as each other,
optionally wherein
the ophthalmic apparatus (100) further includes an image display unit (30) configured to display at least one of the base image and the output image, and an image display control means configured to make the image display unit (30) display at least one of the base image and the output image, and
the arithmetic unit (50) is made to display the output image on the image display unit (30) every time the output image is updated.

14. The program for generating a high dynamic-range image for an ophthalmic apparatus according to claim 13, wherein
based on either one of at least one of values (an R value, a G value, and a B value) out of RGB values in each of pixels of the test subject eye image and the base image, or a conversion value obtained by converting the RGB values with a conversion formula set in advance, the arithmetic unit (50) is made to use an average value of the values or the conversion values of the pixels in each of the divided areas as the identicalness determining numerical value.

15. The program for generating a high dynamic-range image for an ophthalmic apparatus according to claim 11 or 12, wherein
the ophthalmic apparatus (100) further includes an image display unit (30) configured to display at least one of the base image and the output image, and an image display control means configured to make the image display unit (30) display at least one of the base image and the output image, and
the arithmetic unit (50) is made to display the output image on the image display unit (30) every time the output image is updated.
